(19) Europäisches Patentamt
European Patent Office
Office européen des brevets

(11) Veröffentlichungsnummer: **0 282 720 B1**

(12) **EUROPÄISCHE PATENTSCHRIFT**

(45) Veröffentlichungstag der Patentschrift: **29.09.93**

(51) Int. Cl.$^5$: **C08G 77/38**, C08G 77/54

(21) Anmeldenummer: **88101675.2**

(22) Anmeldetag: **05.02.88**

# (54) Polyquaternäre Polysiloxan-Polymere, deren Herstellung und Verwendung in kosmetischen Zubereitungen.

(30) Priorität: **18.02.87 DE 3705121**

(43) Veröffentlichungstag der Anmeldung:
**21.09.88 Patentblatt 88/38**

(45) Bekanntmachung des Hinweises auf die Patenterteilung:
**29.09.93 Patentblatt 93/39**

(84) Benannte Vertragsstaaten:
**BE DE ES FR GB IT NL**

(56) Entgegenhaltungen:
**EP-A- 0 017 121**
**EP-A- 0 166 122**
**FR-A- 1 398 638**
**FR-A- 2 535 730**
**US-A- 4 083 856**
**US-A- 4 390 713**

**PATENT ABSTRACTS OF JAPAN, Band 4, Nr. 106 (C-20)[588], 30. Juli 1980; & JP-A-55 66 506**

(73) Patentinhaber: **Th. Goldschmidt AG**
**Goldschmidtstrasse 100**
**D-45127 Essen(DE)**

(72) Erfinder: **Schaefer, Dietmar, Dr.**
**Milchstrasse 40**
**D-4300 Essen 14(DE)**
Erfinder: **Krakenberg, Manfred**
**Meistersingerstrasse 39**
**D-4300 Essen 13(DE)**

**EP 0 282 720 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige Polysiloxan-Polymere mit quaternären Ammoniumgruppen und Verfahren zur Herstellung dieser Verbindungen. Sie betrifft ferner die Verwendung dieser Polymeren in kosmetischen Zubereitungen.

Es ist bekannt, Organopolysiloxane zur Herstellung von Haarpflegemitteln zu verwenden. In "Chemie und Technologie der Silicone" von Walter Noll, Verlag Chemie, 2. Auflage, 1968, Seite 536, heißt es allerdings, daß die Aufgabe, die Frisur unabhängig von Feuchtigkeitseinflüssen zu erhalten, mit normalen Polydimethylsiloxanolen nicht zu lösen sei. Das Silicon müsse vielmehr mit Hilfe von funktionellen Gruppierungen auf dem Haar fixiert werden.

Aus der DE-AS 14 93 384 sind Organosiloxanverbindungen oder -verbindungsgemische der Formel

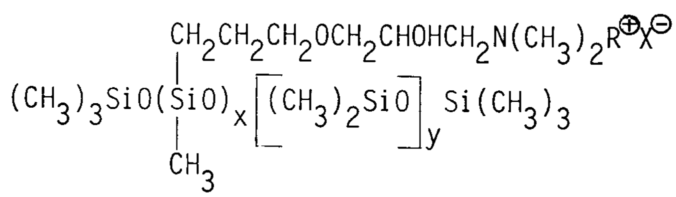

in der R für Wasserstoff oder $CH_3$ und X für Halogen steht und x = 1 bis 10 und y = 0 bis 8,5 sind, wobei y : x nicht größer als 8,5 : 1 ist, bekannt.

Diese Organosiloxane mit quaternären Ammoniumgruppen können dadurch hergestellt werden, daß man ein Epoxysiloxan der Formel

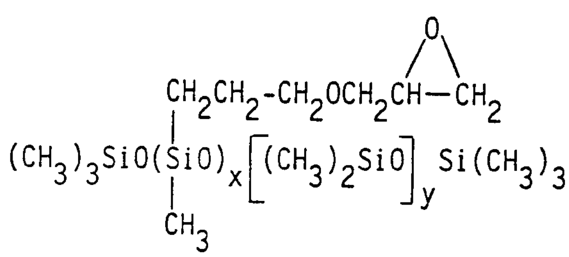

auf an sich bekannte Weise mit Dimethylamin umsetzt und das erhaltene Dimethylaminoorganosiloxan der Formel

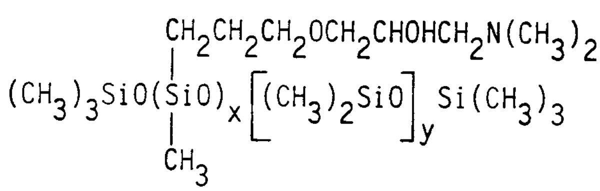

in an sich bekannter Weise mit einem Halogenwasserstoff oder mit einem Methylhalogenid in die quaternäre Ammoniumverbindung der vorgenannten Formel überführt.

Die vorgenannten Organopolysiloxane mit quaternären Ammoniumgruppen können entsprechend der US-PS 4 185 087 für Haarpflegemittel verwendet werden. Dort ist ausgeführt, daß zwar einfache wäßrige Haarwaschmittel das Haar von Schmutz befreien und einen Überschuß an Fett entfernen könnten. Bei den meisten Haarwaschmitteln würde jedoch die Entfettung des Haares so gründlich vorgenommen, daß eine Schädigung des Haares zu beobachten sei. Die Haare würden sich nach der Wäsche elektrostatisch aufladen und deshalb schlecht kämmbar sein.

Der Zusatz von Lanolinderivaten, Glykol, Fettsäureestern oder Proteinen verbessere zwar die Handhabbarkeit des Haares nach dem Waschen, beeinträchtige aber gleichzeitig die Schaumbildung. Die Haare würden etwa klebrig und fühlten sich unnatürlich an.

Die vorbeschriebenen Organopolysiloxane mit quaternären Ammoniumgruppen sollen nach den Angaben der US-PS 4 185 087 diese Nachteile beseitigen und die Kämmbarkeit der gewaschenen Haare, den halt der Frisur, den Glanz der Haare verbessern.

Für die Herstellung der in der DE-AS 14 93 384 beschriebenen Verbindungen geht man von den entsprechenden Methylwasserstoffpolysiloxanen aus. Diese sind im allgemeinen äquilibrierte Gemische, d.h. Siloxangemische, bei denen die Anzahl der Methylwasserstoffsiloxy- und Dimethylsiloxyeinheiten einer statistischen Verteilung entsprechen. Bei Siloxanen mit niedrigem Wert von x sind deshalb stets nicht zu vernachlässigende Anteile an solchen Siloxanen vorhanden, bei denen x = 0 ist. Im Verfahrensendprodukt ist deshalb ein Anteil an unmodifizierten Siliconölen nicht zu vermeiden. Dieser Anteil trägt jedoch nicht zur Verbesserung der Kämmbarkeit des Haares, der Frisur und des Glanzes des Haares bei.

Ein weiterer Nachteil der in der DE-AS 14 93 384 beschriebenen Verbindungen besteht ferner darin, daß die Dimethylsiloxyketten stets durch Methylsiloxygruppen unterbrochen werden, welche seitenständig quaternäre Stickstoffgruppen aufweisen. Der typische Siloxancharakter, der zur Verbesserung der Eigenschaften des Haares erwünscht ist, beruht aber gerade auf der Anwesenheit von Dimethylsiloxyketten. Die Kämmbarkeit und der Glanz des Haares sind deshalb nicht optimal gewährleistet.

Eine ähnliche Lehre ergibt sich aus der EU-PS 0 017 121 (entsprechend DE-OS 29 12 485). Auch hier sind Organopolysiloxane mit quaternären Ammoniumgruppen in Haarwasch- und Haarbehandlungsmitteln zur Verbesserung frisiertechnischen Eigenschaften der Haare beschrieben. Die Verbindungen entsprechen dabei der allgemeinen Formel

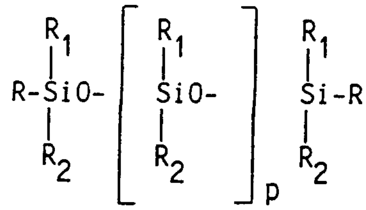

in der $R_1$ und $R_2$ einen Alkylrest mit 1 bis 4 Kohlenstoffatomen oder einen Arylrest, p die Zahlen 0 bis 50 und R die Reste

a) 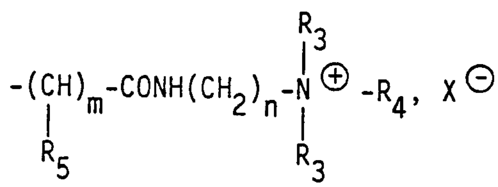

oder

a) 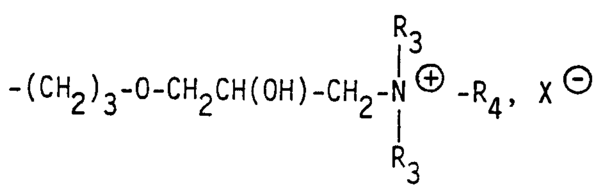

darstellen, wobei $R_3$ einen Alkyl- oder Hydroxy-alkylrest mit 1 bis 3 Kohlenstoffatomen, $R_4$ einen Rest gleich $R_3$ oder Aryl-$CH_2$- oder den Allylrest, $R_5$ Wasserstoff oder den Methylrest, $X^\ominus$ die Anionen $Cl^\ominus$, $Br^\ominus$, $J^\ominus$, $CH_3SO_4^\ominus$ oder $C_2H_5SO_4^\ominus$ und m die
Zahlen 2 bis 10, n die Zahlen 2 bis 4 bedeuten.

Ein Nachteil dieser Verbindungen besteht darin, daß die bei kleinen Werten von p, also relativ niedrigen Molekulargewichten, leicht ausgewaschen werden können, da der organische Charakter der Verbindungen überwiegt und ihre Substantivität gering ist. Bei hohen Molekulargewichten überwiegen zunehmend die Eigenschaften der Dimethylsiloxyeinheiten, während der Einfluß der quaternären Ammoniumgruppen abnimmt. Im Hinblick auf die gewünschten anwendungstechnischen Eigenschaften ist man deshalb gezwungen, einen relativ engen Molekulargewishtsbereich einzuhalten, um eine Ausgewogenheit der gewünschten Eigenschaften zu gewährleisten.

Aus der DE-OS 33 40 708 sind polyquaternäre Polysiloxan-Polymere bekannt, die im wesentlichen aus den folgenden, wiederkehrenden Einheiten der allgemeinen Formel bestehen:

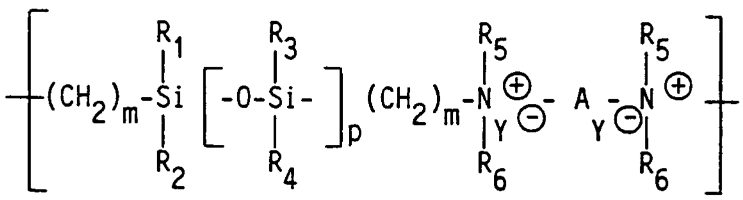

worin

| | |
|---|---|
| A | eine α,ω-Bis-alkylpolysiloxangruppe oder eine lineare oder verzweigte, gesättigte oder ungesättigte Kohlenwasserstoffkette, die bis zu 6 aufeinanderfolgende Kohlenstoffatome enthält und die eine oder mehrere Hydroxygruppe(n) aufweisen und durch ein oder mehrere Sauerstoffatom(e) und/oder durch einen oder mehrere aromatische Ring(e) unterbrochen sein kann, bedeutet; |
| $R_1$, $R_2$, $R_3$ und $R_4$, | die gleich oder verschieden sein können, einen $C_{1-6}$-Alkylrest oder einen Phenylrest bedeuten; |
| $R_5$ | einen Methyl-, Ethyl- oder Hydroxyethylrest bedeutet; |
| $R_6$ | einen $C_{1-18}$-Alkylrest oder einen bivalenten $-CH_2$-Rest bedeutet, wobei im letzteren Fall die beiden Reste $R_6$ miteinander verbunden sind und zusammen mit den beiden Stickstoffatomen und dem Rest A, der eine $-CH_2-CH_2$-Gruppe bedeutet, eine bivalente Piperazinogruppe bilden; oder wobei die Reste $R_5$ und $R_6$ jeweils eine $-CH_2$-Gruppe bedeuten und zusammen mit den beiden Stickstoffatomen und dem Rest A, der eine $CH_2-CH_2$-Gruppe darstellt, einen bivalenten Triethylendiamino-Rest bilden; oder wobei die Reste $R_5$ und $R_6$ miteinander verbunden sind und zusammen mit dem Stickstoffatom, an das sie gebunden sind, einen Heterocyclus, z.B. einen Piperidino-, Morpholino- oder Pyrrolidino-Cyclus, bilden; |
| $Y^-$ | ein $Cl^-$-, $Br^-$-, $CH_3SO_3^-$- oder 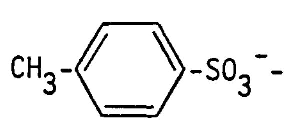 Anion bedeutet; |
| m | eine ganze Zahl von 1 bis 6 bedeutet; und |
| p | eine ganze Zahl oder Dezimalzahl von 1 bis 50 bedeutet. |

Polyquaternäre Polysiloxan-Polymere dieses Typs weisen die oben beschriebenen Nachteile der Verbindungen gemäß DE-OS 29 12 485 nicht auf. Der praktischen Verwendung dieser Verbindungen steht jedoch deren aufwendiges Herstellverfahren entgegen. Die Verbindungen sind in wirtschaftlich nicht vertretbaren Ausbeuten von ≦ 60% der Theorie herstellbar.

Schließlich sei noch auf die veröffentlichte europäische Patentanmeldung 0 095 238 verwiesen, die eine Zusammensetzung betrifft, die im wesentlichen aus folgenden Bestandteilen besteht:

A) einem Siloxan der allgemeinen Formel

$$R_aX_{3-a}Si(OSiX_2)_n(OSiX_bR_{2b})_mOSiX_{3-a}R_a$$

wobei R nur aufgabenhaft als eine funktionelle Gruppe angegeben ist, welche die Haftung am Haar bewirkt, z.B. eine Amino-, Carbonsäure- oder quaternäre Ammoniumgruppe. X ist ein Wasserstoffrest oder eine Phenyl-, Hydroxyl- oder gesättigte Kohlenwasserstoffgruppe mit 1 bis 8 Kohlenstoffatomen, a

hat einen Wert von 0 bis 3, b hat einen Wert von 0 bis 1 und n + m hat einen Wert von 1 bis 1999, wobei n einen Wert von 0 bis 2000 und m einen Wert von 1 bis 2000 hat,

B) einem Tensid,

C) einem Zusatz zur Verbesserung der Gefrier/Auftau-Stabilität und

D) Wasser.

Der Erfindung liegt die Aufgabe zugrunde, polyquaternäre Polysiloxan-Polymere zu finden, welche sich mit wesentlich höheren Ausbeuten, als es nach dem Stand der Technik möglich ist, herstellen lassen und darüber hinaus das gewünschte Eigenschaftsprofil aufweisen, welches durch die Kombination folgender Eigenschaften gegeben ist: gute Kämmbarkeit der behandelten Haare, ansprechender Glanz der Haare, erhöhte Substantivität und hierdurch bedingt verminderte Auswaschbarkeit der Verbindungen auf der Oberfläche der Haare, hohe antistatische Wirksamkeit der Verbindungen, gute physiologische Verträglichkeit der Verbindungen gegenüber den Haaren sowie dem Haarboden.

Überraschenderweise wurde gefunden, daß diese Eigenschaften bei Polysiloxan-Polymeren mit quaternären Ammoniumgruppen zu finden sind, die aus cyclischen Verbindungen der allgemeinen Formel

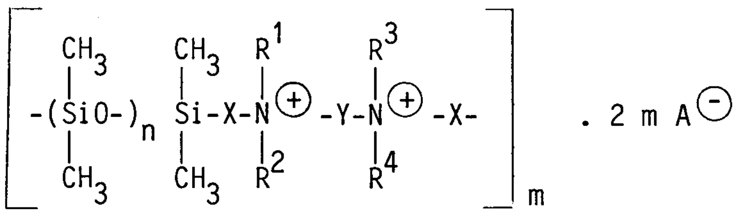

I

und/oder aus linearen Verbindungen der allgemeinen Formel

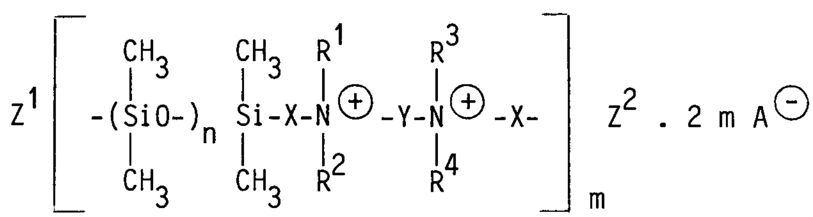

II

bestehen, wobei

X ein zweiwertiger Kohlenwasserstoffrest mit mindestens 4 Kohlenstoffatomen ist, der eine Hydroxylgruppe aufweist und der durch ein Sauerstoffatom unterbrochen sein kann,

Y ein zweiwertiger Kohlenwasserstoffrest mit mindestens 2 Kohlenstoffatomen ist, der eine Hydroxylgruppe aufweisen und der durch ein Sauerstoff- oder Stickstoffatom unterbrochen sein kann,

$Z^1$ ein H, OH, ein Alkyl- oder ein Alkoxyrest ist, oder die Bedeutung eines Kohlenwasserstoffrestes mit mindestens 4 Kohlenstoffatomen hat, der eine oder mehrere Hydroxylgruppe(n) aufweist und durch ein oder mehrere Sauerstoffatome unterbrochen sein kann oder die Bedeutung des Restes

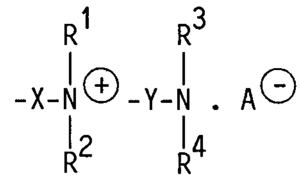

oder

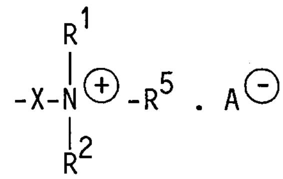

hat, wobei $R^5$ ein Alkylrest mit 1 bis 20 Kohlenstoffatomen ist,

$Z^2$ die Gruppe

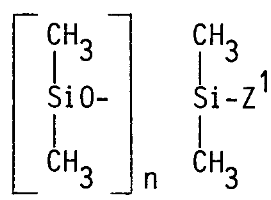

bedeutet,

$R^1$, $R^2$, $R^3$, $R^4$ gleich oder verschieden sind und Alkylreste mit 1 bis 4 Kohlenstoffatomen oder Benzylreste bedeuten oder jeweils die Reste $R^1$ und $R^3$ oder $R^2$ und $R^4$ Bestandteile eines verbrückenden Alkylenrestes sein können,

$A^{\ominus}$ ein anorganisches oder organisches Anion ist,

n = 5 bis 200 und

m = ganze Zahl $\geqq 1$.

Die Polymeren entsprechen in ihrem Aufbau dem Typ AB, wobei der Block A durch die Formel

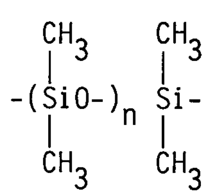

und der Block B durch die Formel

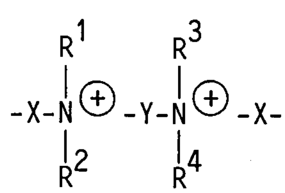

wiedergegeben wird.

Ein besonderer Vorteil dieses polymeren Typus besteht darin, daß bei gegebenem Verhältnis der Blöcke A und B das Molekulargewicht praktisch beliebig eingestellt werden kann, ohne daß sich das Verhältnis der Dimethylsiloxyeinheiten zu der Anzahl der Einheiten mit quaternären Stickstoffatomen verändert. Dies ist deshalb von Bedeutung, da eine Reihe von anwendungstechnischen Eigenschaften, wie die Viskosität der Lösungen der Polymeren, deren Pflegeeigenschaften für das Haar und weitere Eigenschaften, von dem Molekulargewicht abhängig sind und gerade in der Kosmetik Produkte mit höheren Molekulargewichten besonders erwünscht sind.

In der Formel I ist X ein zweiwertiger Kohlenwasserstoffrest mit mindestens 4 Kohlenstoffatomen, der eine Hydroxylgruppe aufweist und der durch ein Sauerstoffatom unterbrochen sein kann. Vorzugsweise weist der zweiwertige Kohlenwasserstoffrest 4 bis 10 Kohlenstoffatome auf. Besonders bevorzugt sind die folgenden Reste

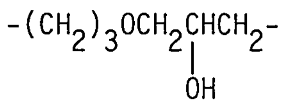 , 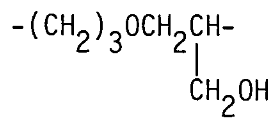 ,

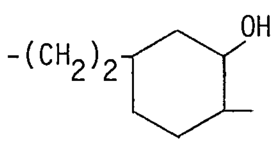 , 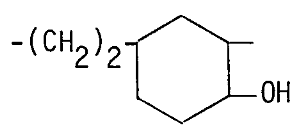 ,

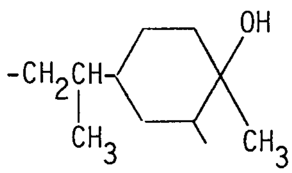 , 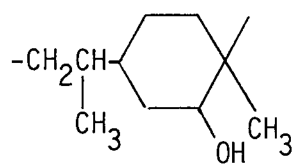

Beispiele weiterer Reste X sind

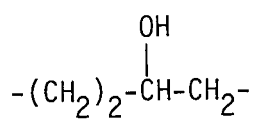 , 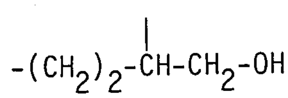 ,

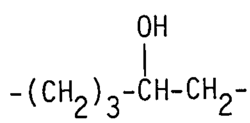 , 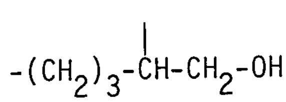 ,

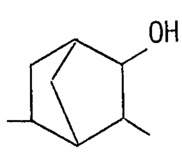 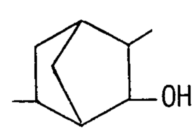

Y ist ein zweiwertiger Kohlenwasserstoffrest mit mindestens 2 Kohlenstoffatomen, der eine Hydroxylgruppe aufweisen und der durch ein Sauerstoff- oder Stickstoffatom unterbrochen sein kann. Bevorzugt sind zweiwertige Kohlenwasserstoffreste mit 2 bis 6 Kohlenstoffatomen, insbesondere Reste der Formel $-(CH_2)_o-$, wobei o 2 bis 6 ist.

Weitere Beispiele geeigneter Reste Y sind

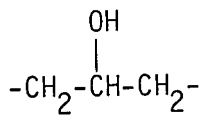 ,

$-CH_2-CH_2-O-CH_2-CH_2-$, $-CH_2-O-CH_2-$ ,

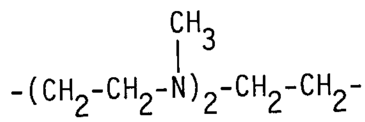

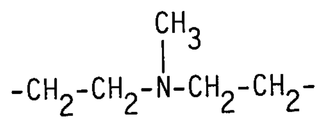 ,

Die Reste $R^1$, $R^2$, $R^3$ und $R^4$ können gleich oder verschieden sein und die Bedeutung von Alkylresten mit 1 bis 4 Kohlenstoffatomen oder die von Benzylresten haben. Die Reste $R^1$ und $R^3$ sowie die Reste $R^2$ und $R^4$ können Bestandteil eines verbrückenden Alkylenrestes sein. Der Polymerblock B kann dabei folgende Struktur annehmen:

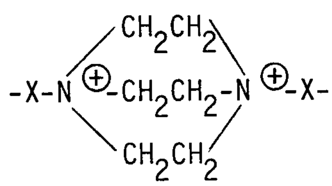

Vorzugsweise sind die Reste $R^1$, $R^2$, $R^3$ und $R^4$ Methylreste.

Beispiele von Endgruppen $Z^1$ sind

H-, HO-, $H_3C-CH_2O-$, $(CH_3)_2CHO-$,

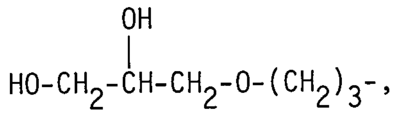

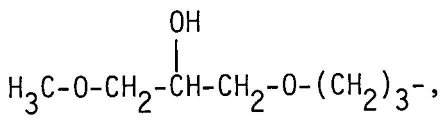

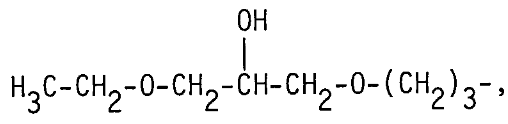

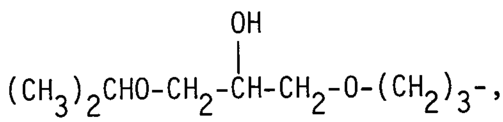

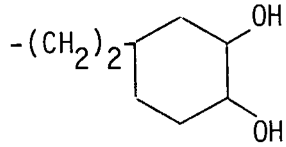

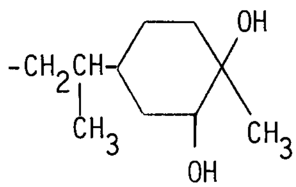

,

$-C_8H_{17}$

$A^{\ominus}$ ist ein beliebiges anorganisches oder organisches Anion. Bei Verwendung der erfindungsgemäßen neuen Polymeren als Bestandteil von Haarpflegemitteln ist darauf zu achten, daß das Anion von einer physiologisch verträglichen Säure herrührt. Beispiele geeigneter Anionen sind Acetationen, Chloridionen, Bromidionen, Hydrogensulfationen,Sulfationen.

n definiert die Anzahl der Dimethylsiloxyeinheitem im Block A und ist eine Zahl von 5 bis 200. Da es sich bei der angegebenen Formel I um eine allgemeine Formel handelt, stellen diese Zahlenwerte Mittelwerte dar.

m definiert die Anzahl der Polymerblöcke AB und ist gleich oder größer 1.

Beispiele erfindungsgemäßer Polymerer sind

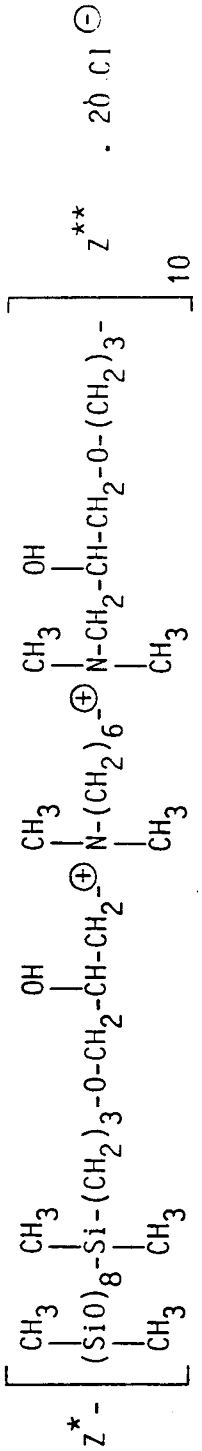

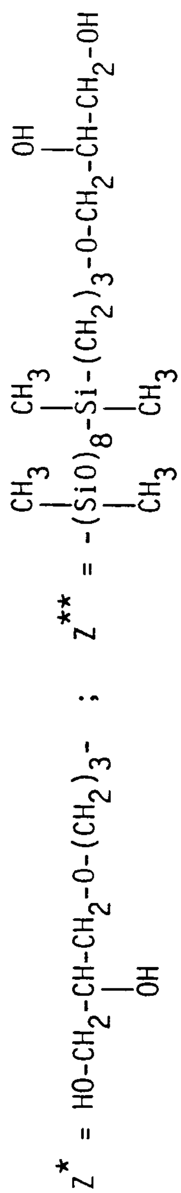

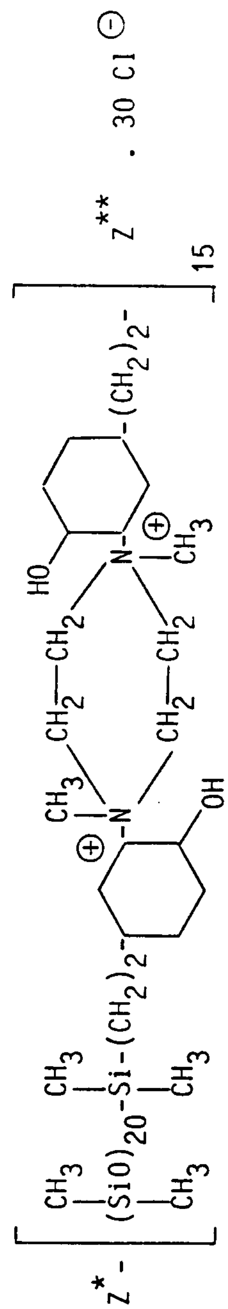

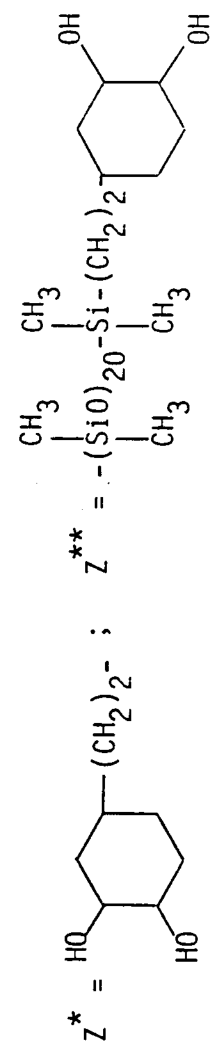

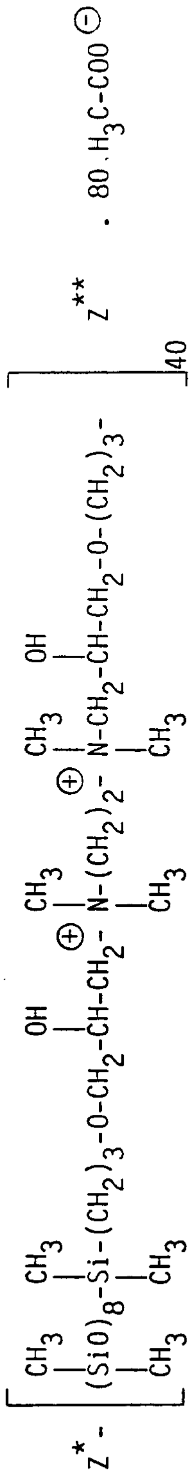

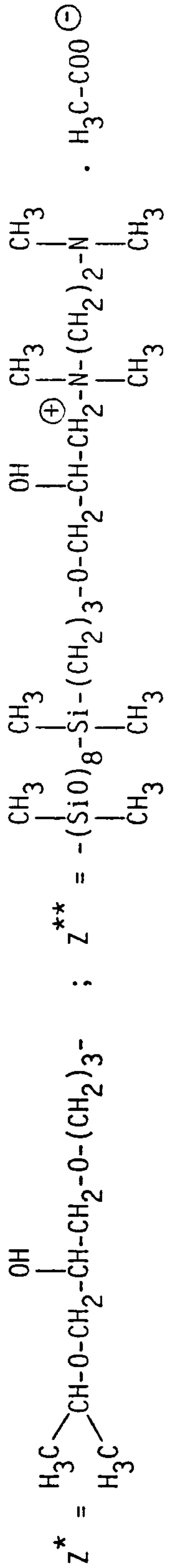

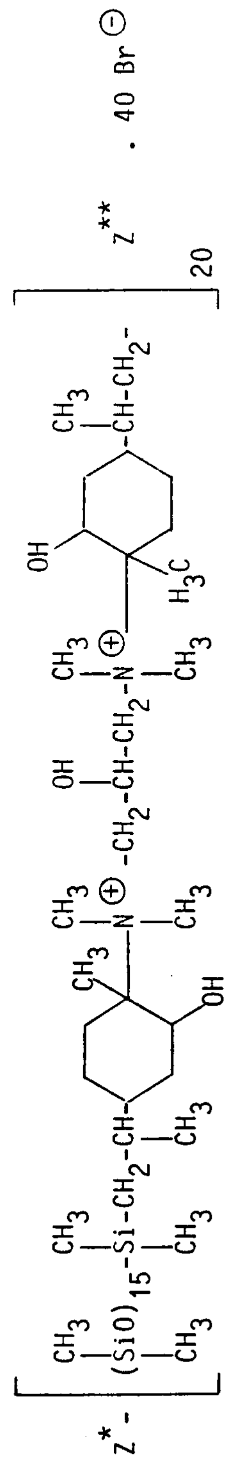

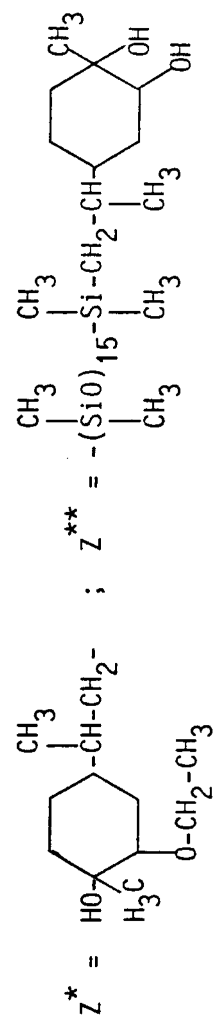

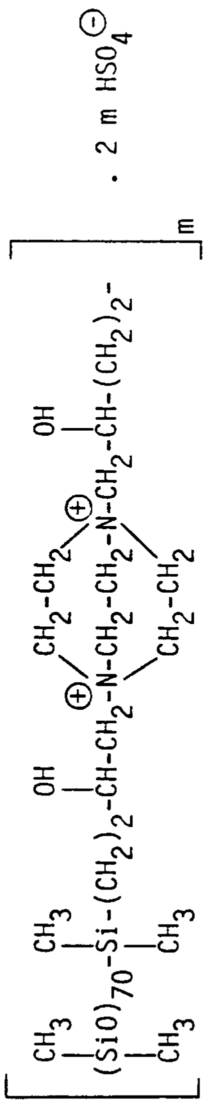

m > 1

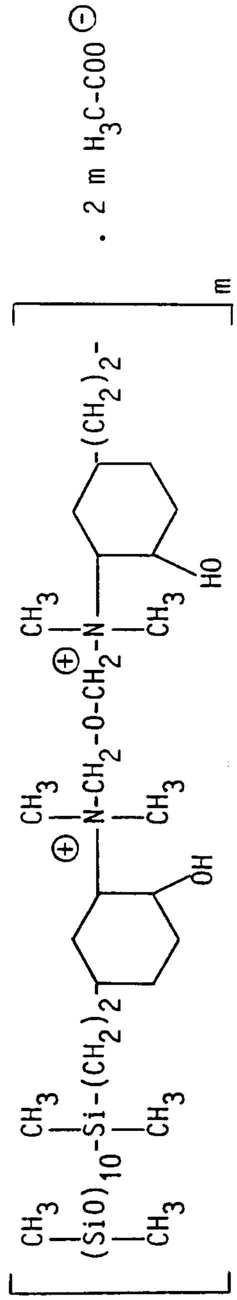

m > 1

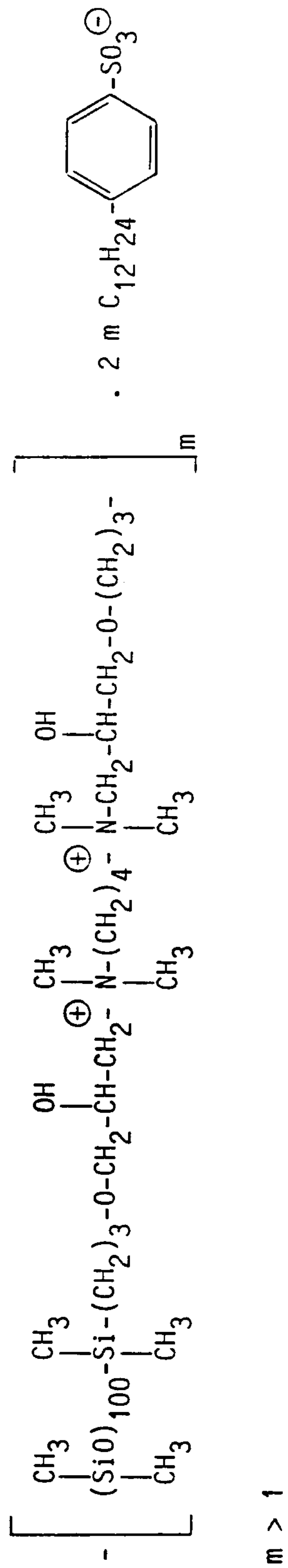

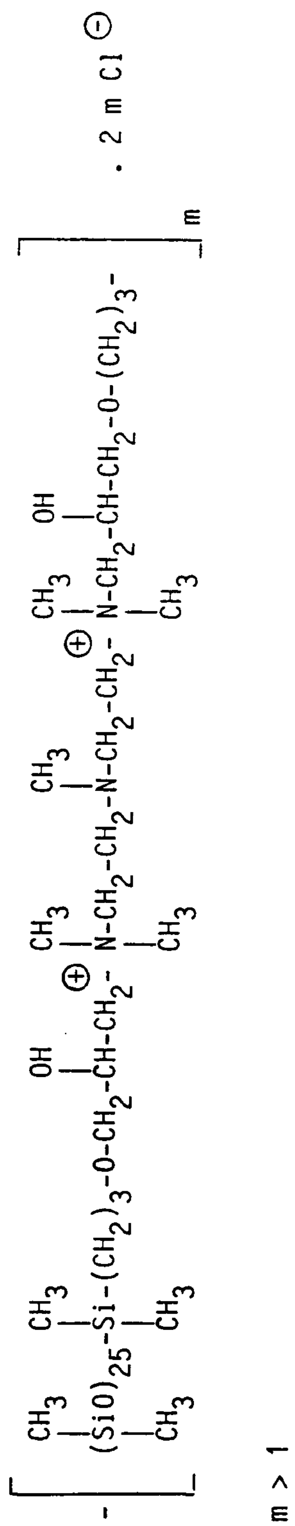

Die erfindungsgemäßen Produkte sind viskose bis hochviskose, ölige bis pastenartige, farblose bis schwach gelb oder rötlich gefärbte Produkte. Die Löslichkeit der erfindungsgemäßen Polymeren wird durch das Verhältnis der Dimethylsiloxyeinheiten und der Zahl der quaternären Ammoniumgruppen und durch das Molekulargewicht der Verbindungen bestimmt.

Für die Anwendung in der Kosmetik, speziell in Haarpflegemitteln, sind im allgemeinen Produkte bevorzugt, die in Wasser oder in wassermischbaren Hilfslösungsmitteln, wie ein- oder mehrwertigen Alkoholen, löslich sind.

Ein weiterer Gegenstand der vorliegenden Erfindung besteht in der Herstellung der erfindungsgemäßen Verbindungen. Das erfindungsgemäße Verfahren ist dadurch gekennzeichnet, daß man zunächst α,ω-Wasserstoffpolysiloxane der allgemeinen Formel

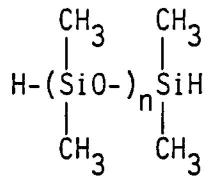

mit, bezogen auf SiH-Gruppen, äquimolaren Mengen eines Epoxides, welches endständig eine olefinische Bindung hat, wobei das Epoxid mindestens 4 Kohlenstoffatome aufweist und zusätzlich eine nichtcyclische Ethergruppe enthalten kann, in Gegenwart eines Hydrosilylierungskatalysators bei Temperaturen von 50 bis 150 °C in an sich bekannter Weise umsetzt und das so erhaltene Reaktionsprodukt mit einem ditertiären Diamin der allgemeinen Formel

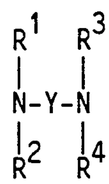

in Gegenwart von 2 Moläquivalenten Säure HA, bezogen auf Diamin, bei Temperaturen von 40 bis 120 °C in solchen Mengen umsetzt, daß jeder Epoxidgruppe eine tertiäre Amingruppe entspricht.

Das erfindungsgemäße Verfahren ist ein in an sich bekannter Weise ablaufendes Verfahren. Das Verfahren geht von Wasserstoffpolysiloxanen mit endständigen SiH-Gruppen aus, wobei die Anzahl der Dimethylsiloxyeinheiten entsprechend dem gewünschten Endprodukt ausgewählt wird. An die endständigen SiH-Gruppen der Wasserstoffpolysiloxane werden nun äquimolare Mengen eines Epoxides angelagert, wobei das Epoxid endständig eine olefinische Doppelbindung aufweist, mindestens 4 Kohlenstoffatome hat und zusätzlich eine nichtcyclische Ethergruppe enthalten kann. Die Anlagerung der endständigen olefinisch ungesättigten Gruppe an die SiH-Gruppe erfolgt in Gegenwart eines Hydrosilylierungskatalysators bei Temperaturen von 50 bis 150 °C. Als Hydrosilylierungskatalysator können die hierfür bekannten Platinkatalysatoren, wie z.B. hexachloroplatinsäure oder cis-Dichlorodiaminplatin, verwendet werden.

Vorzugsweise werden als Epoxide mit endständiger olefinischer Gruppe solche der Formel

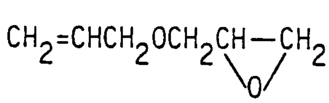 , 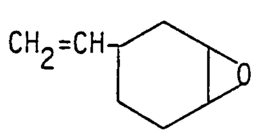 , 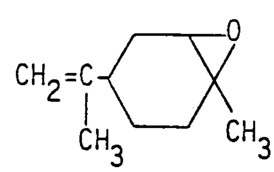

eingesetzt.

Dieser erste Reaktionsschritt wird durch folgende Gleichung verdeutlicht:

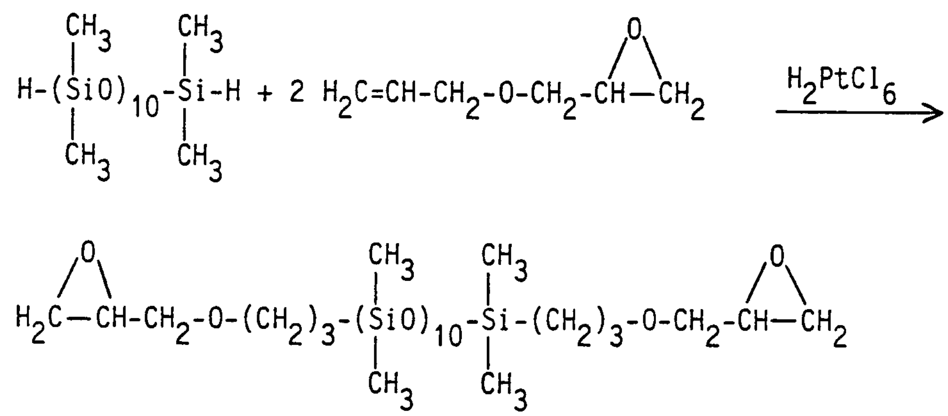


Das so erhaltene Reaktionsprodukt wird nun mit einem ditertiären Diamin der allgemeinen Formel

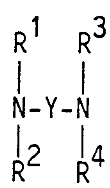

in Gegenwart von 2 Moläquivalenten der Säure HA, bezogen auf Diamin, bei Temperaturen von 40 bis 120 °C in solchen Mengen umgesetzt, daß jeder Epoxidgruppe eine tertiäre Amingruppe entspricht.

Vorzugsweise verwendet man als ditertiäre Diamine solche der Formel

$(CH_3)_2N\text{-}(CH_2)_o\text{-}N(CH_3)_2$

wobei o = 2 bis 6 ist.

Die erste Stufe des zweistufigen Herstellungsverfahrens wird vorzugsweise ohne Verwendung eines Lösungsmittels durchgeführt. Die zweite Stuffe der Umsetzung erfolgt vorzugsweise in wäßriger oder wäßrig-alkoholischer Lösung. Die maximale Umsetzungstemperature der Reaktion der zweiten Stufe ergibt sich deshalb im allgemeinen aus der Rückflußtemperatur des verwendeten Lösungsmittels. Sie soll jedoch 120 °C nicht überschreiten.

Die Ausbeuten bei dem erfindungsgemäßen Verfahren betragen im allgemeinen mindestens 90, meist jedoch 95 % der Theorie und liegen zum Teil noch darüber.

Ein weiterer Gegenstand der Erfindung liegt in der Verwendung der erfindungsgemäßen Verbindungen in der Kosmetik, insbesondere, wie eingangs ausgeführt, in der Verwendung der Verbindungen in Zubereitungen zur Pflege der Haare. Die erfindungsgemäßen Verbindungen weisen die geforderte Kombination der Eigenschaften, die eingangs aufgezählt worden sind, auf.

Verfahren zur Herstellung der erfindungsgemäßen Verbindungen und die Eigenschaften dieser Verbindungen sind in den folgenden Beispielen näher beschrieben.

Beispiel 1

Herstellung eines erfindungsgemäßen Polymeren der allgemeinen Formel

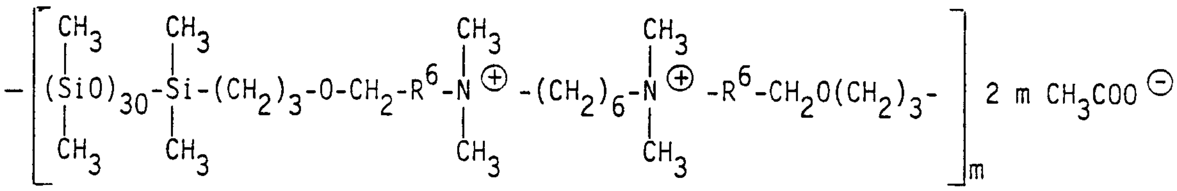

$R^6$ = 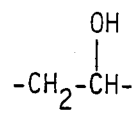 oder 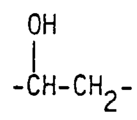 , m > 1

In einem 0,5 l Vierhalskolben, versehen mit Rührer, Tropftrichter, Thermometer und Rückflußkühler, werden 30 g (0,26 Mol) Allylglycidether zusammen mit 0,022 g (7,4 × $10^{-5}$ Mol) $(NH_3)_2PtCl_2$ unter Stickstoffatmosphäre auf 115 °C erwärmt. Hierzu werden innerhalb 15 Minuten 228 g (0,1 Mol) eines α,ω-Wasserstoffsiloxans der allgemeinen Formel

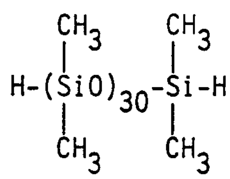

getropft. Das Reaktionsgemisch wird weitere 3 Stunden bei 115 °C gerührt, anschließend wird der überschüssige Allylglycidether bei 0,2 bar und 100 °C abdestilliert.

Erhalten: 246 g (0,098 Mol; 98 %) eines α,ω-Diepoxysiloxans der allgemeinen Formel

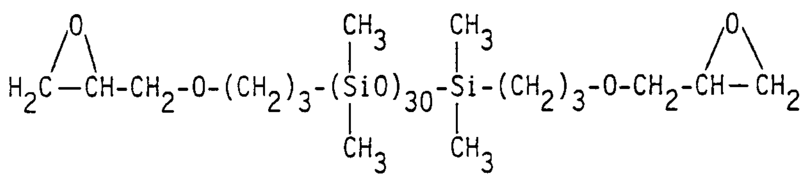

Zur weiteren Umsetzung werden in einem 1 l Vierhalskolben, versehen mit Rührer, Tropftrichter, Thermometer und Rückflußkühler, 14,1 g (0,098 Mol) N,N,N',N'-Tetramethyl-1,4-butandiamin mit 40 g $H_2O$ vorgelegt und bei 20 °C mit 12 g (0,2 Mol) Essigsäure versetzt. Nach 30 Minuten wird auf 50 °C erwärmt, das oben erhaltene α,ω-Diepoxysiloxan zugetropft und nach Zugabe von 100 ml Isopropanol auf Rückflußtemperature erwärmt und 6 Stunden gerührt. Das Wasser/Isopropanol-Gemisch wird bei 100 °C und 0,2 bar abdestilliert.

Erhalten:

268 g polyquaternäres Polysiloxan-Polymer; gelbes bis rötlich gefärbtes Produkt, bei Raumtemperatur kaum fließfähig;
quaternärer Stickstoff gef.: 0,9 % theor.: 1,0 %.

Beispiel 2

Herstellung eines Polymeren der allgemeinen Formel

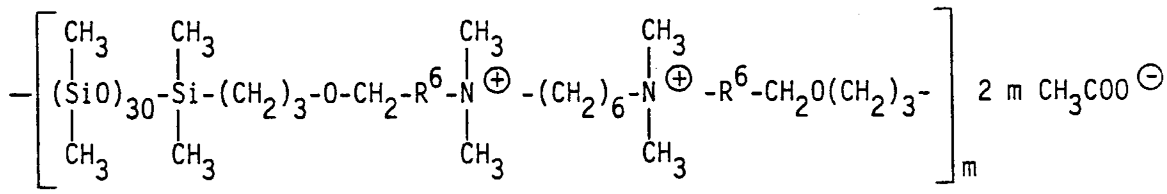

$m > 1$

Durchführung wie in Beispiel 1 beschrieben mit N,N,N′,N′-Tetramethyl1,6-hexandiamin anstelle von N,N,N′, N′-Tetramethyl-1,4-butandiamin.

Ansatz:

| | |
|---|---|
| N,N,N',N'-Tetramethyl-1,6-hexandiamin: | 16,8 g (0,098 Mol) |
| Essigsäure: | 12 g (0,2 Mol) |
| α,ω-Diepoxysiloxan aus Beispiel 1: | 245 g (0,098 Mol) |
| Wasser: | 40 g |
| Isopropanol: | 100 g |

Erhalten:

272 g polyquaternäres Polysiloxan-Polymer; gelbes bis rötlich gefärbtes Produkt, bei Raumtemperatur kaum fließfähig;
quaternärer Stickstoff gef.: 1,0 % theor.: 1,0 %.

Beispiel 3

Herstellung eines Polymeren der allgemeinen Formel

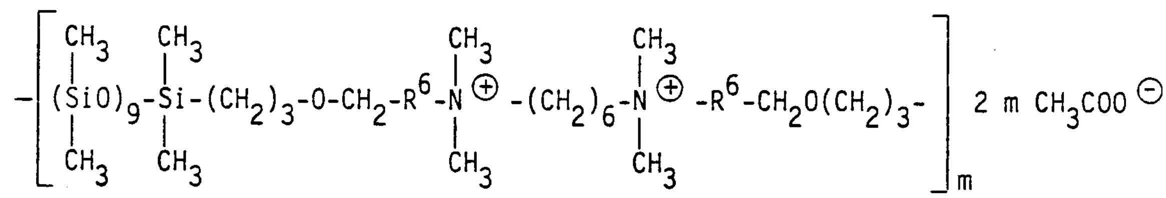

$m > 1$

In einem 0,5 l Vierhalskolben, versehen mit Rührer, Tropftrichter, Thermometer und Rückflußkühler, werden 57 g (0,5 Mol) Allylglycidether zusammen mit 0,022 g ($7{,}4 \times 10^{-5}$ Mol) $(NH_3)_2PtCl_2$ unter Stickstoffatmosphäre auf 115 °C erwärmt. Hierzu werden innerhalb 15 Minuten 145 g (0,2 Mol) eines α,ω-Wasserstoffsiloxans der allgemeinen Formel

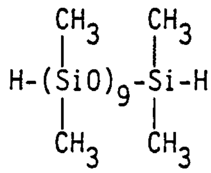

getropft. Das Reaktionsgemisch wird weitere 3 Stunden bei 115 °C gerührt, anschließend wird der überschüssige Allylglycidether bei 0,2 bar und 100 °C abdestilliert.

Erhalten: 182 g (0,19 Mol ; 95 %) eines $\alpha,\omega$-Diepoxysiloxans der allgemeinen Formel

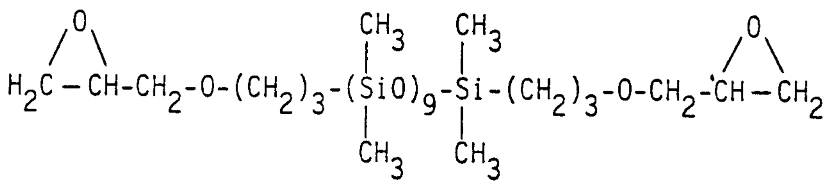

Zur weiteren Umsetzung werden in einem 1 l Vierhalskolben, versehen mit Rührer, Tropftrichter, Thermometer und Rückflußkühler, 32,7 g (0,19 Mol) N,N,N′,N′-Tetramethyl-1,6-butandiamin mit 40 g $H_2O$ vorgelegt und bei 20 °C mit 24 g (0,4 Mol) Essigsäure versetzt. Nach 30 Minuten wird auf 50 °C erwärmt, das oben erhaltene $\alpha,\omega$-Diepoxysiloxan zugetropft und nach Zugabe von 100 ml Isopropanol auf Rückflußtemperatur erwärmt und 5 Stunden gerührt. Das Wasser/Isopropanol-Gemisch wird bei 100 °C und 0,2 bar abdestilliert.

Erhalten:

236 g polyquaternäres Polysiloxan-Polymer; gelbes bis rötlich gefärbtes Produkt, bei Raumtemperatur kaum fließfähig;
quaternärer Stickstoff gef.: 2,2 % theor.: 2,2 %.

Beispiel 4

Herstellung Überprüfung von Haarbehandlungsmitteln unter Verwendung der in den Beispielen 1 bis 3 hergestellten polyquaternären Polysiloxan-Polymeren (%-Angaben in Gew.-%)

| Konditioniershampoo I | | | Konditioniershampoo II | |
|---|---|---|---|---|
| | Zusammensetzung: | | | |
| A | Tego®-Betain L 7 1) | 2 % | Tego®-Betain L 7 1) | 3 % |
| A | ANTIL® 141 LIQUID 2) | 3 % | ANTIL® 141 LIQUID 2) | 3 % |
| B | Polyquat aus Beisp. 1 | 2 % | Polyquat aus Beisp. 2 | 2 % |
| B | 1,2-Propylenglykol | 4 % | 1,2-Propylenglykol | 4 % |
| C | Natriumlaurylethersulfat | 10 % | Triethanolaminlaurylsulfat | 12 % |
| E | Natriumchlorid | 3,5 % | - | |
| D | Wasser | 75,5 % | Wasser | 76 % |

Zur Herstellung werden die Bestandteile in der aufgeführten Reihenfolge (A→E) zusammengegeben. Jede Mischung muß vor Zugabe weiterer Komponenten klar gelöst sein.

Cremespülung

| | | |
|---|---|---|
| A | TEGINACID® X 3) | 6 % |
| A | Cetylalkohol | 0,5 % |
| B | Polyquat aus Beisp. 3 | 2 % |
| B | Wasser | 91,5 % |

Zur Herstellung werden A und B zusammengegeben, homogenisiert und unter Rühren abgekühlt.

1) Tego®-Betain L 7 = Cocamidopropyl-Betain (1-alkylamino-3-dimethylammonium-propan-3-carboxymethyl-betain)

2) ANTIL® 141 LIQUID ist ein flüssiges Verdickungsmittel auf der Basis eines nichtionogenen Fettsäure-polyalkylenglykolesters

3) TEGINACID® X ist ein O/W-Emulgator auf der Basis einer Mischung von Glycerinmono/distearaten mit Polyglykolfettalkoholethern

In der praktischen Anwendung beim halbseitigen Vergleichstest auf menschlichem Haar ergibt sich gegenüber Shampooformulierungen bzw. Cremespülungen mit polyquaternären Siloxanen aus dem Stand der Technik sowie gegenüber Konditioniershampoos und Cremespülungen des Marktes auf Basis rein organischer Polyquats eine verbesserte Naß- und Trockenkämmbarkeit, verbesserter Glanz sowie verringerte elektrostatische Aufladung der behandelten Haare.

**Patentansprüche**

**Patentansprüche für folgende Vertragsstaaten : BE, DE, FR, GB, IT, NL**

**1.** Polysiloxan-Polymere mit quaternären Ammoniumgruppen, die aus cyclischen Verbindungen der allgemeinen Formel

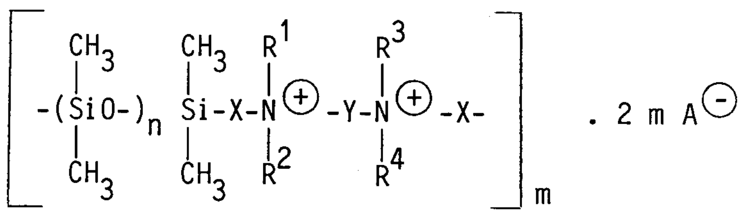

I

und/oder aus linearen Verbindungen der allgemeinen Formel

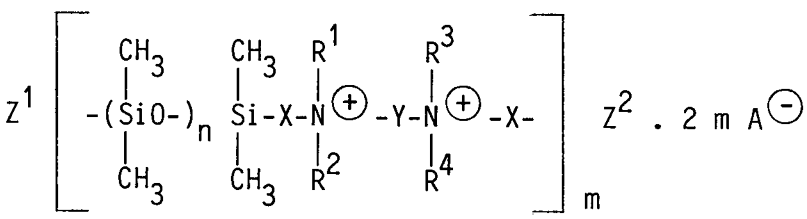

II

bestehen, wobei

| | |
|---|---|
| X | ein Zweiwertiger Kohlenwasserstoffrest mit mindestens 4 Kohlenstoffatomen ist, der eine Hydroxylgruppe aufweist und der durch ein Sauerstoffatom unterbrochen sein kann, |
| Y | ein zweiwertiger Kohlenwasserstoffrest mit mindestens 2 Kohlenstoffatomen ist, der eine Hydroxylgruppe aufweisen und der durch ein Sauerstoff- oder Stickstoffatom unterbrochen sein kann, |
| $Z^1$ | ein H, OH, ein Alkyl- oder ein Alkoxyrest ist, oder die Bedeutung eines Kohlenwasserstoffrestes mit mindestens 4 Kohlenstoffatomen hat, der eine oder mehrere Hydroxylgruppe(n) aufweist und durch ein oder mehrere Sauerstoffatome unterbrochen sein kann oder die Bedeutung des Restes |

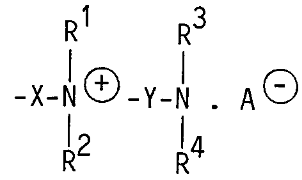

oder

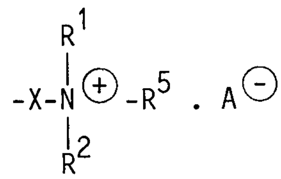

hat, wobei $R^5$ ein Alkylrest mit 1 bis 20 Kohlenstoffatomen ist,

$Z^2$ die Gruppe

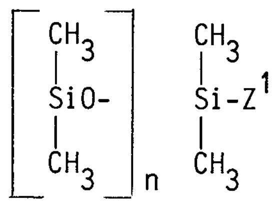

bedeutet,

$R^1$, $R^2$, $R^3$, $R^4$ gleich oder verschieden sind und Alkylreste mit 1 bis 4 Kohlenstoffatomen oder Benzylreste bedeuten oder jeweils die Reste $R^1$ und $R^3$ oder $R^2$ und $R^4$ Bestandteile eines verbrückenden Alkylenrestes sein können,

$A^{\ominus}$ ein anorganisches oder organisches Anion ist,

n = 5 bis 200 und

m = ganze Zahl ≧ 1.

2. Polymere nach Anspruch 1, dadurch gekennzeichnet, daß X ein Rest der Gruppe

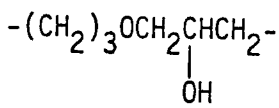 , 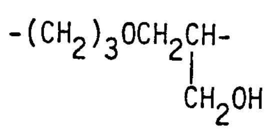 ,

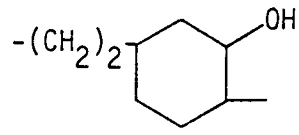 , 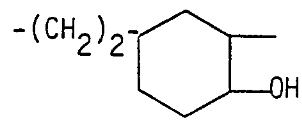 ,

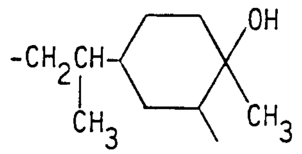 , 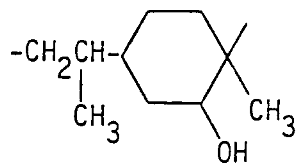

ist.

3. Polymere nach Anspruch 1 oder 2, dadurch gekennzeichnet, daß Y ein Rest $-(CH_2)_o-$ ist, wobei o = 2 bis 6 ist.

4. Polymere nach einem oder mehreren der vorhergehenden Ansprüche, dadurch gekennzeichnet, daß $R^1$, $R^2$, $R^3$ und $R^4$ Methylreste sind.

5. Verfahren zur Herstellung der Polymeren nach Anspruch 1 bis 4, dadurch gekennzeichnet, daß man zunächst α,ω-Wasserstoffpolysiloxane der allgemeinen Formel

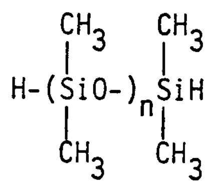

mit, bezogen auf SiH-Gruppen, äquimolaren Mengen eines Epoxides, welches endständig eine olefinische Bindung hat, wobei das Epoxid mindestens 4 Kohlenstoffatome aufweist und zusätzlich eine nichtcyclische Ethergruppe enthalten kann, in Gegenwart eines Hydrosilylierungskatalysators bei Temperaturen von 50 bis 150 °C in an sich bekannter Weise umsetzt und das so erhaltene Reaktionsprodukt mit einem ditertiären Diamin der allgemeinen Formel

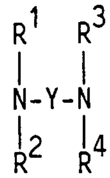

in Gegenwart von 2 Moläquivalenten Säure HA, bezogen auf Diamin, bei Temperaturen von 40 bis 120 °C in solchen Mengen umsetzt, daß jeder Epoxidgruppe eine tertiäre Amingruppe entspricht.

6. Verfahren nach Anspruch 5, dadurch gekennzeichnet, daß man die α,ω-Wasserstoffpolysiloxane mit einem Epoxid aus der Gruppe

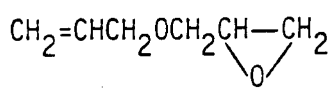 , 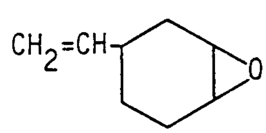 , 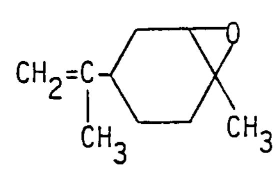

umsetzt.

**7.** Verfahren nach Anspruch 5, dadurch gekennzeichnet, daß man das in der ersten Stufe erhaltene Epoxidgruppen aufweisende Polysiloxan mit Aminen der Formel

$(CH_3)_2N\text{-}(CH_2)_o\text{-}N(CH_3)_2$

wobei o = 2 bis 6 ist, umsetzt.

**8.** Verfahren nach Anspruch 5, dadurch gekennzeichnet, daß man die Umsetzung des in der ersten Stufe erhaltenen Epoxidgruppen aufweisenden Polysiloxans mit dem ditertiären Diamin in wäßriger oder wäßrig-alkoholischer Lösung durchführt.

**9.** Verwendung der Verbindungen der Ansprüche 1 bis 4 in kosmetischen Zubereitungen, insbesondere in Zubereitungen zur Pflege der Haare.

**Patentansprüche für folgenden Vertragsstaat : ES**

**1.** Verfahren zur Herstellung von Polysiloxan-Polymere mit quaternären Ammoniumgruppen, die aus cyclischen Verbindungen der allgemeinen Formel

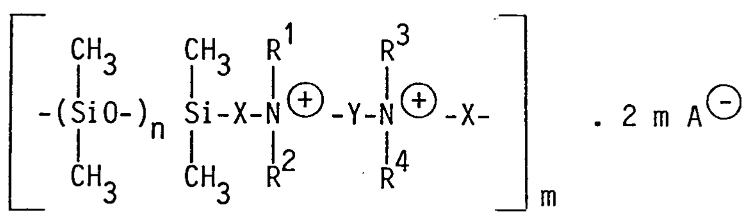

I

und/oder aus linearen Verbindungen der allgemeinen Formel

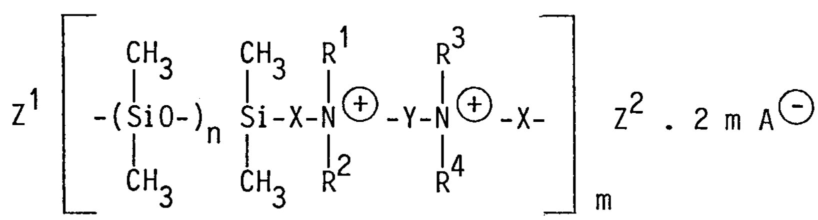

II

bestehen, wobei

| | |
|---|---|
| X | ein zweiwertiger Kohlenwasserstoffrest mit mindestens 4 Kohlenstoffatomen ist, der eine Hydroxylgruppe aufweist und der durch ein Sauerstoffatom unterbrochen sein kann, |
| Y | ein zweiwertiger Kohlenwasserstoffrest mit mindestens 2 Kohlenstoffatomen ist, der eine Hydroxylgruppe aufweisen und der durch ein Sauerstoff- oder Stickstoffatom unterbrochen sein kann, |
| $Z^1$ | ein H, OH, ein Alkyl- oder ein Alkoxyrest ist, oder die Bedeutung eines Kohlenwasserstoffrestes mit mindestens 4 Kohlenstoffatomen hat, der eine oder mehrere Hydroxylgruppe(n) aufweist und durch ein oder mehrere Sauerstoffatome unterbrochen sein kann oder die Bedeutung des Restes |

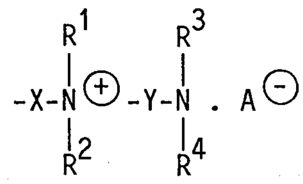

oder

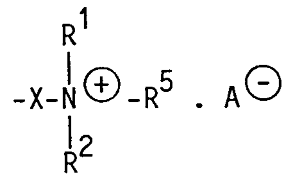

hat, wobei $R^5$ ein Alkylrest mit 1 bis 20 Kohlenstoffatomen ist,

$Z^2$ die Gruppe

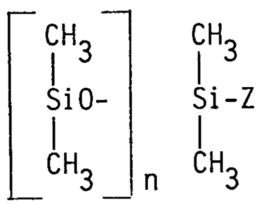

bedeutet,

$R^1$, $R^2$, $R^3$, $R^4$ gleich oder verschieden sind und Alkylreste mit 1 bis 4 Kohlenstoffatomen oder Benzylreste bedeuten oder jeweils die Reste $R^1$ und $R^3$ oder $R^2$ und $R^4$ Bestandteile eines verbrückenden Alkylenrestes sein können,

$A^{\ominus}$ ein anorganisches oder organisches Anion ist,

n = 5 bis 200 und

m = ganze Zahl ≧ 1,

dadurch gekennzeichnet, daß man zunächst $\alpha,\omega$-Wasserstoffpolysiloxane

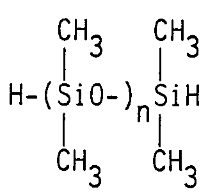

mit, bezogen auf SiH-Gruppen, äquimolaren Mengen eines Epoxides, welches endständig eine olefinische Bindung hat, wobei das Epoxid mindestens 4 Kohlenstoffatome aufweist und zusätzlich eine nichtcyclische Ethergruppe enthalten kann, in Gegenwart eines Hydrosilylierungskatalysators bei Temperaturen von 50 bis 150 °C in an sich bekannter Weise umsetzt und das so erhaltene Reaktionsprodukt mit einem ditertiären Diamin der allgemeinen Formel

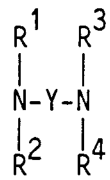

in Gegenwart von 2 Moläquivalenten Säure HA, bezogen auf Diamin, bei Temperaturen von 40 bis 120 °C in solchen Mengen umsetzt, daß jeder Epoxidgruppe eine tertiäre Amingruppe entspricht.

2. Verfahren nach Anspruch 1, dadurch gekennzeichnet, daß man die α,ω-Wasserstoffpolysiloxane mit einem Epoxid aus der Gruppe

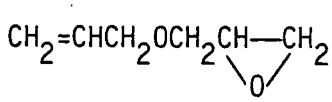 , 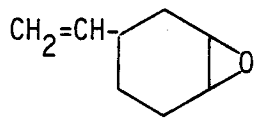 , 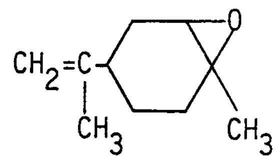

umsetzt.

3. Verfahren nach Anspruch 1, dadurch gekennzeichnet, daß man das in der ersten Stufe erhaltene Epoxidgruppen aufweisende Polysiloxan mit Aminen der Formel

$(CH_3)_2N-(CH_2)_o-N(CH_3)_2$

wobei o = 2 bis 6 ist, umsetzt.

4. Verfahren nach Anspruch 1, dadurch gekennzeichnet, daß man die Umsetzung des in der ersten Stufe erhaltenen Epoxidgruppen aufweisenden Polysiloxans mit dem ditertiären Diamin in wäßriger oder wäßrig-alkoholischer Lösung durchführt.

5. Verwendung der gemäß Ansprüchen 1 bis 4 hergestellten Verbindungen in kosmetischen Zubereitungen, insbesondere in Zubereitungen zur Pflege der Haare.

**Claims**
**Claims for the following Contracting States : BE, DE, FR, GB, IT, NL**

1. Polysiloxane polymers containing quaternary ammonium groups, which comprise cyclic compounds of the general formula

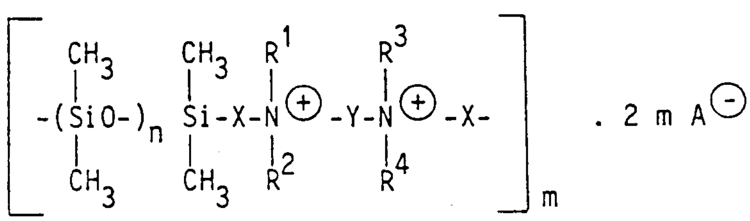 I

and/or linear compounds of the general formula

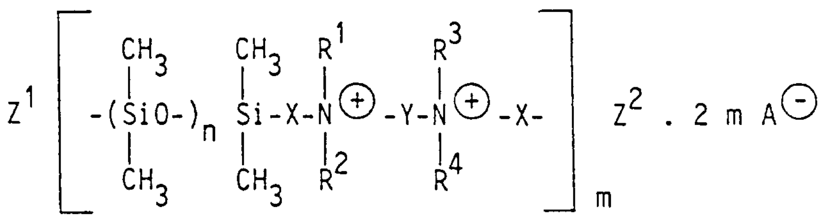

II

where

X is a divalent hydrocarbon radical having at least 4 carbon atoms which contains a hydroxyl group and may be interrupted by an oxygen atom,

Y is a divalent hydrocarbon radical having at least 2 carbon atoms which contains a hydroxyl group and may be interrupted by an oxygen or nitrogen atom,

$Z^1$ is an H, OH, alkyl or alkoxy radical or is a hydrocarbon radical having at least 4 carbon atoms which contains one or more hydroxyl group(s) and may be interrupted by one or more oxygen atoms, or is a

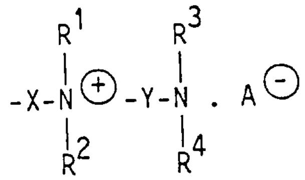

or

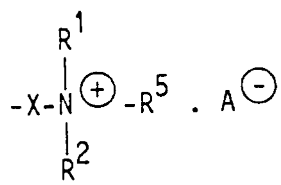

radical where $R^5$ is an alkyl radical having 1 to 20 carbon atoms

$Z^2$ is the

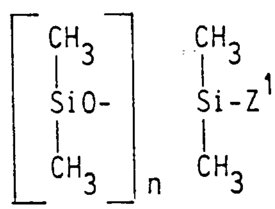

group,

$R^1$, $R^2$, $R^3$ and $R^4$ are identical or different and are alkyl radicals having 1 to 4 carbon atoms or benzyl radicals, or in each case the radicals $R^1$ and $R^3$ or $R^2$ and $R^4$ may be constituents of a bridging alkylene radical,

$A^{\ominus}$ is an inorganic or organic anion,

n is from 5 to 200, and

m is an integer $\geqq 1$.

2. Polymers according to Claim 1, characterised in that X is a radical from the group consisting of

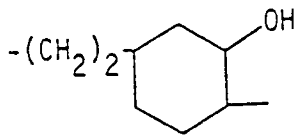 , 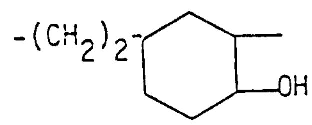 ,

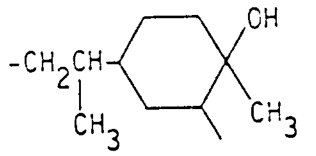 , 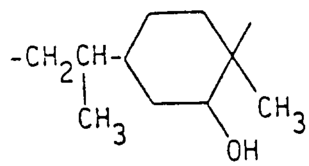

3. Polymers according to Claim 1 or 2, characterised in that Y is a $-(CH_2)_o-$ radical where o is from 2 to 6.

4. Polymers according to one or more of the preceding claims, characterised in that $R^1$, $R^2$, $R^3$ and $R^4$ are methyl radicals.

5. Process for the preparation of polymers according to Claims 1 to 4, characterised in that first α,ω-hydropolysiloxanes of the general formula

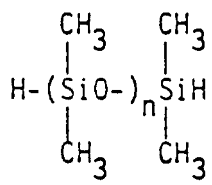

are reacted in a manner known per se with, based on SiH groups, equimolar amounts of an epoxide which contains a terminal olefinic bond, where the epoxide has at least 4 carbon atoms and may additionally contain an acyclic ether group, in the presence of a hydrosilylation catalyst at temperatures of from 50 to 150 °C, and the resultant reaction product is reacted with a ditertiary diamine of the general formula

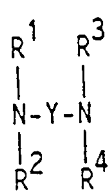

in the presence of 2 mol equivalents of an acid HA, based on diamine, at temperatures of from 40 to 120 °C in such amounts that one tertiary amino group corresponds to each epoxide group.

6. Process according to Claim 5, characterised in that the α,ω-hydropolyslloxanes are reacted with an epoxide from the group consisting of

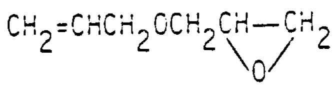 , 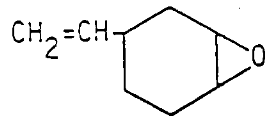 , 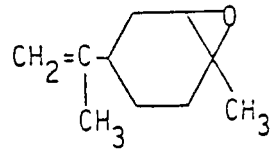

7. Process according to Claim 5, characterised in that the polysiloxane containing epoxide groups which is obtained in the first step is reacted with amines of the formula

$(CH_3)_2N\text{-}(CH_2)_o\text{-}N(CH_3)_2$

where o is from 2 to 6.

8. Process according to Claim 5, characterised in that the reaction of the polysiloxane containing epoxide groups which is obtained in the first step with the ditertiary diamine is carried out in aqueous or aqueous-alcoholic solution.

9. Use of the compounds of Claims 1 to 4 in cosmetic preparations, in particular in hair-care preparations.

**Claims for the following Contracting State : ES**

1. Process for the preparation of polysiloxane polymers containing quaternary ammonium groups, which comprise cyclic compounds of the general formula

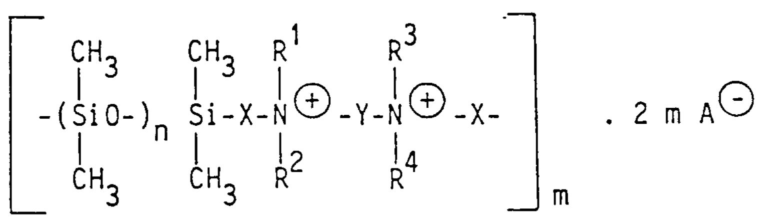 I

and/or linear compounds of the general formula

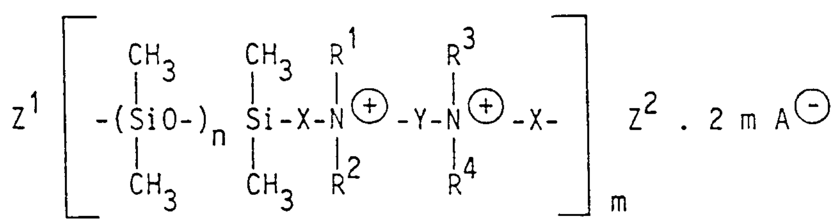 II

where

| | |
|---|---|
| X | is a divalent hydrocarbon radical having at least 4 carbon atoms which contains a hydroxyl group and may be interrupted by an oxygen atom, |
| Y | is a divalent hydrocarbon radical having at least 2 carbon atoms which contains a hydroxyl group and may be interrupted by an oxygen or nitrogen atom, |
| $Z^1$ | is an H, OH, alkyl or alkoxy radical or is a hydrocarbon radical having at least 4 carbon atoms which contains one or more hydroxyl group(s) and may be interrupted by one or more oxygen atoms, or is a |

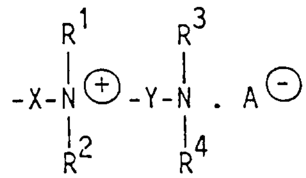

or

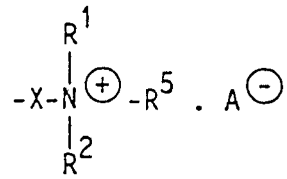

radical where $R^5$ is an alkyl radical having 1 to 20 carbon atoms,

$Z^2$ is the

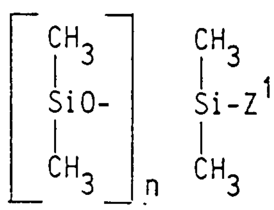

group,

$R^1$, $R^2$, $R^3$ and $R^4$ are identical or different and are alkyl radicals having 1 to 4 carbon atoms or benzyl radicals, or in each case the radicals $R^1$ and $R^3$ or $R^2$ and $R^4$ may be constituents of a bridging alkylene radical,

$A^{\ominus}$ is an inorganic or organic anion,

n is from 5 to 200, and

m is an integer $\geqq 1$,

characterised in that first $\alpha,\omega$-hydropolysiloxanes of the general formula

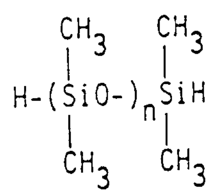

are reacted in a manner known per se with, based on SiH groups, equimolar amounts of an epoxide which contains a terminal olefinic bond, where the epoxide has at least 4 carbon atoms and may additionally contain an acyclic ether group, in the presence of a hydrosilylation catalyst at temperatures of from 50 to 150 °C, and the resultant reaction product is reacted with a ditertiary diamine of the general formula

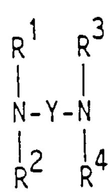

in the presence of 2 mol equivalents of an acid HA, based on diamine, at temperatures of from 40 to

120 °C in such amounts that one tertiary amino group corresponds to each epoxide group.

2. Process according to Claim 1, characterised in that α,ω-hydropolysiloxanes are reacted with an epoxide from the group consisting of

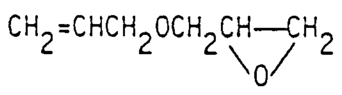

,

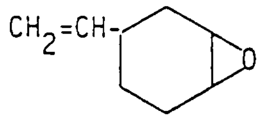

,

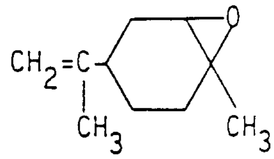

3. Process according to Claim 1, characterised in that the polysiloxane containing epoxide groups which is obtained in the first step is reacted with amines of the formula

$(CH_3)_2N-(CH_2)_o-N(CH_3)_2$

where o is from 2 to 6.

4. Process according to Claim 1, characterised in that the reaction of the polysiloxane containing epoxide groups which is obtained in the first step with the ditertiary diamine is carried out in aqueous or aqueous-alcoholic solution.

5. Use of the compounds prepared according to Claims 1 to 4 in cosmetic preparations, in particular in hair-care preparations.

**Revendications**
**Revendications pour les Etats contractants suivants :BE, DE, FR, GB, IT, NL**

1. Polymères de polysiloxanes contenant des groupes d'ammonium quaternaire, qui sont formés de composés cycliques de formule générale :

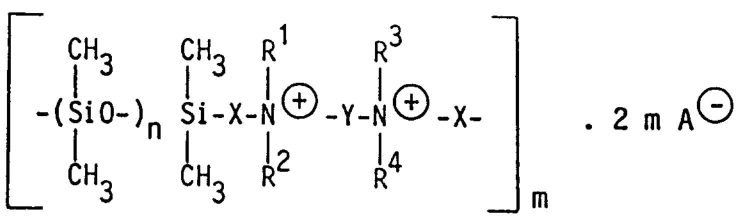

I

et/ou de composés linéaires de formule générale :

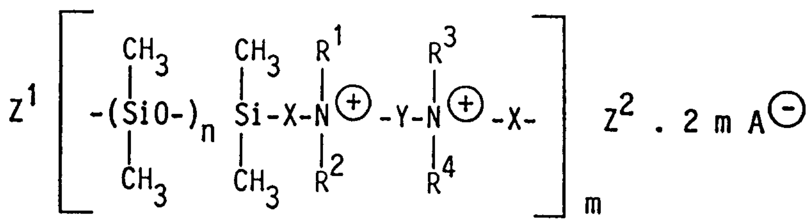

II

où

X représente un reste hydrocarbure bivalent comportant au moins 4 atomes de carbone, qui comporte un groupe hydroxyle et peut être interrompu par un atome d'oxygène,

Y représente un reste hydrocarbure bivalent comportant au moins 2 atomes de carbone, qui peut comporter un groupe hydroxyle et être interrompu par un atome

d'oxygène ou d'azote,

$Z^1$ représente H, OH ou un reste alkyle ou alcoxy, ou bien a la signification d'un reste hydrocarbure comportant au moins 4 atomes de carbone, qui comporte un ou plusieurs groupes hydroxyles et peut être interrompu par un ou plusieurs atomes d'oxygène, ou bien a la signification du reste :

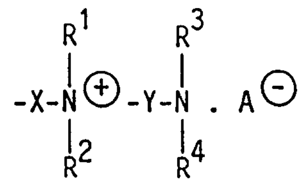

ou

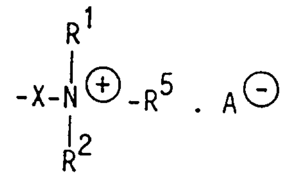

où $R^5$ représente un reste alkyle comportant de 1 à 20 atomes de carbone,

$Z^2$ représente le groupe

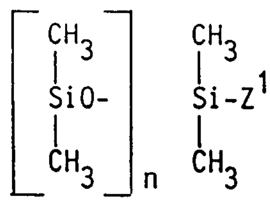

$R^1$, $R^2$, $R^3$, $R^4$ sont identiques ou différents les uns des autres et représentent des restes alkyles comportant de 1 à 4 atomes de carbone ou des restes benzyles, ou bien les restes $R^1$ et $R^3$ ou $R^2$ et $R^4$ peuvent être, dans chaque cas, des constituants d'un reste alkyle pontant,

$A^{\ominus}$ représente un anion inorganique ou organique,

n = 5 à 200, et

m = un nombre entier ≧ 1.

**2.** Polymères selon la revendication 1, caractérisés en ce que X est un reste du groupe :

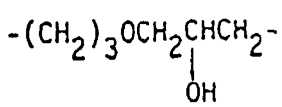 , 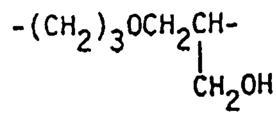 ,

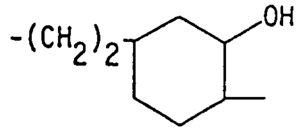 , 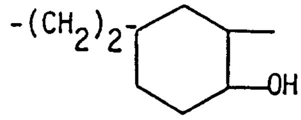 ,

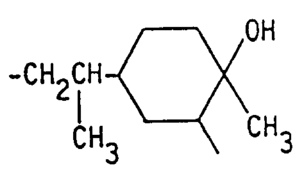 , 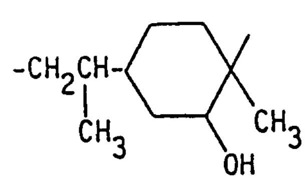 .

**3.** Polymères selon la revendication 1 ou 2, caractérisés en ce que Y est un reste $-(CH_2)_o-$ où $o = 2$ à 6.

**4.** Polymères selon une ou plusieurs des revendications précédentes, caractérisés en ce que $R^1$, $R^2$, $R^3$ et $R^4$ sont des restes méthyle.

**5.** Procédé de préparation des polymères selon l'une des revendications 1 à 4, caractérisé en ce qu'on fait d'abord réagir des $\alpha,\omega$-hydrogénopolysiloxanes de formule générale :

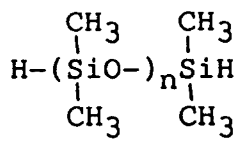

avec, par rapport aux groupes SiH, des quantités équimolaires d'un époxyde qui comporte une liaison oléfinique en fin de chaîne, cet époxyde comportant au moins 4 atomes de carbone et pouvant contenir, en outre, un groupe d'éther non cyclique, en présence d'un catalyseur d'hydrosilylation, à une température comprise entre 50 et 150 °C, de manière connue en soi, et on fait réagir le produit de réaction ainsi obtenu avec une diamine ditertiaire de formule générale :

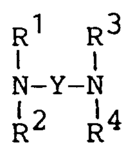

en présence de 2 équivalents molaires d'acide HA, par rapport à la diamine, à une température comprise entre 40 et 120 °C, en des quantités telles qu'à chaque groupe d'époxyde correspond un groupe d'amine tertiaire.

**6.** Procédé selon la revendication 5, caractérisé en ce qu'on fait réagir les $\alpha,\omega$-hydrogénopolysiloxanes avec un époxyde choisi dans le groupe constitué de :

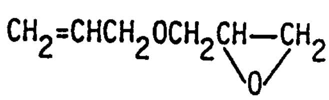 , 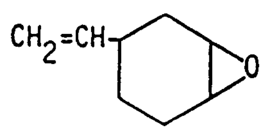 , 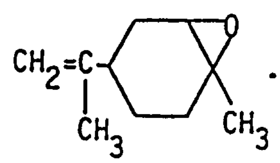 .

**7.** Procédé selon la revendication 5, caractérisé en ce qu'on fait réagir le polysiloxane comportant des groupes époxydes, obtenu lors de la première étape, avec des amines de formule :

$(CH_3)_2N-(CH_2)_o-N(CH_3)_2$

dans laquelle $o = 2$ à 6.

**8.** Procédé selon la revendication 5, caractérisé en ce qu'on effectue la réaction du polysiloxane comportant des groupes époxydes, obtenu lors de la première étape, avec l'amine ditertiaire, en solution aqueuse ou aqueuse-alcoolique.

**9.** Utilisation des composés selon les revendications 1 à 4, dans des préparations cosmétiques, en particulier dans des préparations pour les soins des cheveux.

**Revendications pour l'Etat contractant suivant : ES**

1. Procédé de préparation de polymères de polysiloxanes contenant des groupes d'ammonium quaternaire, qui sont formés de composés cycliques de formule générale :

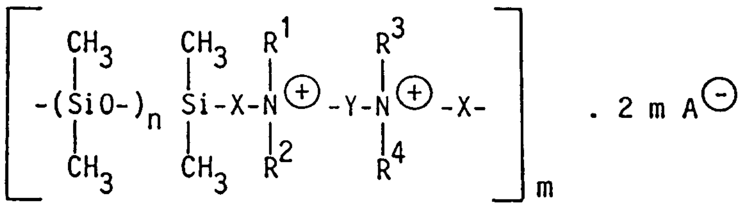

I

et/ou de composés linéaires de formule générale :

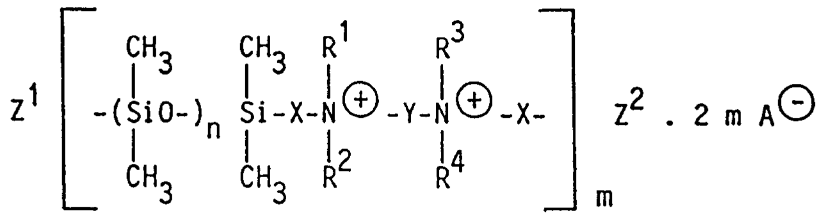

II

où

X représente un reste hydrocarbure bivalent comportant au moins 4 atomes de carbone, qui comporte un groupe hydroxyle et peut être interrompu par un atome d'oxygène,

Y représente un reste hydrocarbure bivalent comportant au moins 2 atomes de carbone, qui peut comporter un groupe hydroxyle et être interrompu par un atome d'oxygène ou d'azote,

$Z^1$ représente H, OH ou un reste alkyle ou alcoxy, ou bien a la signification d'un reste hydrocarbure comportant au moins 4 atomes de carbone, qui comporte un ou plusieurs groupes hydroxyles et peut être interrompu par un ou plusieurs atomes d'oxygène, ou bien a la signification du reste :

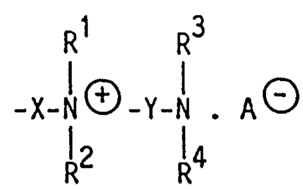

ou

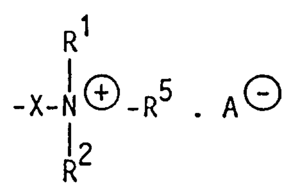

où $R^5$ représente un reste alkyle comportant de 1 à 20 atomes de carbone,

$Z^2$ représente le groupe

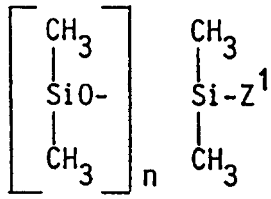

| | |
|---|---|
| $R^1$, $R^2$, $R^3$, $R^4$ | sont identiques ou différents les uns des autres et représentent des restes alkyles comportant de 1 à 4 atomes de carbone ou des restes benzyles, ou bien les restes $R^1$ et $R^3$ ou $R^2$ et $R^4$ peuvent être, dans chaque cas, des constituants d'un reste alkyle pontant, |
| $A^{\ominus}$ | représente un anion inorganique ou organique, |
| n | = 5 à 200, et |
| m | = un nombre entier ≧ 1, |

caractérisé en ce qu'on fait d'abord réagir des α,ω-hydrogénopolysiloxanes de formule générale :

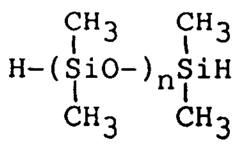

avec, par rapport aux groupes SiH, des quantités équimolaires d'un époxyde qui comporte une liaison oléfinique en fin de chaîne, cet époxyde comportant au moins 4 atomes de carbone et pouvant contenir, en outre, un groupe d'éther non cyclique, en présence d'un catalyseur d'hydrosilylation, à une température comprise entre 50 et 150 °C, de manière connue en soi, et on fait réagir le produit de réaction ainsi obtenu avec une diamine ditertiaire de formule générale :

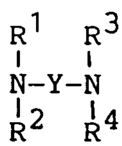

en présence de 2 équivalents molaires d'acide HA, par rapport à la diamine, à une température comprise entre 40 et 120 °C, en des quantités telles qu'à chaque groupe d'époxyde correspond un groupe d'amine tertiaire.

**2.** Procédé selon la revendication 1, caractérisé en ce qu'on fait réagir les α,ω-hydrogénopolysiloxanes avec un époxyde choisi dans le groupe constitué de :

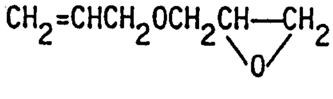 , 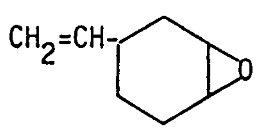 , 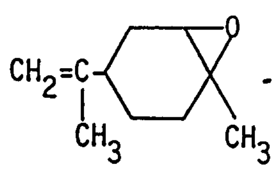 .

**3.** Procédé selon la revendication 1, caractérisé en ce qu'on fait réagir le polysiloxane comportant des groupes époxydes, obtenu lors de la première étape, avec des amines de formule :

$(CH_3)_2N-(CH_2)_o-N(CH_3)_2$

dans laquelle o = 2 à 6.

**4.** Procédé selon la revendication 1, caractérisé en ce qu'on effectue la réaction du polysiloxane comportant des groupes époxydes, obtenu lors de la première étape, avec l'amine ditertiaire, en solution aqueuse ou aqueuse-alcoolique.

**5.** Utilisation des composés selon les revendications 1 à 4, dans des préparations cosmétiques, en particulier dans des préparations pour les soins des cheveux.